# EUROPÄISCHE PATENTSCHRIFT

(11) **EP 0 929 812 B1**
(45) Veröffentlichungstag und Bekanntmachung des Hinweises auf die Patenterteilung: **20.11.2002**
(21) Anmeldenummer: 97942937.0
(22) Anmeldetag: 17.09.1997
(51) Int. Cl.: G01N 33/68, G01N 33/74, C07K 14/64

(54) **DIAGNOSEHILFE SOWIE VERFAHREN ZUM NACHWEIS DER TRÄCHTIGKEIT VON WIEDERKÄUERN**
DIAGNOSTIC AGENT AND METHOD TO DETERMINE PREGNANCY IN RUMINANTS
AUXILIAIRE DE DIAGNOSTIC ET PROCEDE POUR LA DETERMINATION DE LA GRAVIDITE DE RUMINANTS

(30) Priorität: 27.09.1996 DE 19641378
(43) Veröffentlichungstag der Anmeldung: 21.07.1999
(73) Patentinhaber: IHF INSTITUT FÜR HORMON- UND FORTPFLANZUNGSFORSCHUNG GmbH, 22529 Hamburg (DE)
(72) Erfinder: IVELL, Richard, D-20257 Hamburg (DE)
(74) Vertreter: UEXKÜLL & STOLBERG
(86) Internationale Anmeldenummer: EP9705075
(87) Internationale Veröffentlichungsnummer: WO98013693

(56) Entgegenhaltungen:
- M.J. FIELDS ET AL.: "Chemistry of bovine relaxin" ADVANCES IN EXPERIMENTAL MEDICINE AND BIOLOGY, Bd. 143, 1982, NEW YORK NY USA, Seiten 191-207, XP002053014
- L.H. LARKIN ET AL.: "In vitro analysis of antisera to relaxin" ACTA ENDOCRINOLOGICA, Bd. 92, Nr. 3, 1979, COPENHAGEN DK, Seiten 568-576, XP002053015
- M.J. FIELDS ET AL.: "Evidence for relaxin in corpora lutea of late pregnant cows." ENDOCRNOLOGY, Bd. 107, Nr. 4, 1980, WASHINGTON DC USA, Seiten 869-876, XP002053016

## Beschreibung

Die vorliegende Erfindung betrifft Diagnosehilfen sowie Verfahren zum Nachweis der Trächtigkeit von Wiederkäuern, welche auf dem bei Wiederkäuern nachweisbaren Relaxin-ähnlichen Faktor basieren. Ferner werden Antikörper gegen den Relaxin-ähnlichen Faktor von Wiederkäuern sowie gegen Fragmente und/oder aktive Derivate desselben mit gleicher Immunogenität bereitgestellt.

Derzeit existieren mehrere immunologische Verfahren zur Bestimmung der Gravidität zahlreicher Säugetiere. So ist beispielsweise bekannt, daß Schwangerschaften bei Frauen durch Nachweis von spezifischen Antikörpern gegen das humane Choriongonadotropin (HCG) im Harn der zu untersuchenden Personen zuverlässig nachgewiesen werden können, wobei insbesondere Hämagglutinations-Hemmungstests, Latexagglutinations-Hemmungstests oder radioimmunologische Nachweise von β-HCG angewendet werden. Darüber hinaus besteht die Möglichkeit, die Gravidität bei mehreren Säugerarten durch Bestimmung des vor allem in Uterus und Plazenta während der Schwangerschaft gebildeten Gelbkörperhormons

Relaxin nachzuweisen, das während der Gravidität sehr früh ein bestimmtes Niveau übersteigt, welches demgegenüber im normalen nicht-schwangeren Zyklus nie überschritten wird. Es konnte sogar gezeigt werden, daß ein spezifischer Anstieg des Relaxins im peripheren Serum während eines sehr frühen Stadiums, nämlich noch vor der Einnistung der Blastozyste und noch vor einem eindeutigen positiven HCG-Test stattfindet (D. R. Stewart et al., Relaxin in the peri-implantation period, J. Clin. Endocrinol. Metab., 70: 1771-1773 (1990)). Demgemäß ist vorgeschlagen worden, alternative Schwangerschaftstests auf Grundlage des Nachweises von Relaxin bereitzustellen. Beispielsweise werden im US-Patent Nr. 5 108 897 Verfahren beschrieben zur Bestimmung der Trächtigkeit bei Hündinnen, bei denen die in bestimmten Körperflüssigkeiten oder Geweben der Tiere im Falle einer Gravidität nachweisbaren Relaxinwerte gemessen werden.

Kürzlich ist neben Relaxin ein weiteres Peptidhormon mit der Bezeichnung Relaxin-ähnlicher Faktor (kurz: RLF) identifiziert worden (E. Bullesbach et al., A novel Leydig cell cDNA-derived protein is a relaxin-like factor, J. Biol. Chem., 270: 16011-16015 (1995)). Während Relaxin im wesentlichen die Funktion zugeschrieben wird, die Physiologie von weiblichen Tieren der meisten Säugerarten zu regulieren, scheint der RLF nach derzeitigem Wissensstand bei den meisten Arten ausschließlich in den Leydig-Zellen der männlichen Keimdrüsen exprimiert zu werden, da in diesem Gewebe größere Mengen an spezifischer mRNA nachgewiesen werden konnten (W. Pusch et al., Molecular cloning and expression of the relaxin-like factor from the mouse testis, Endocrinology, 137 (1996, im Druck); I.M. Adham et al., Cloning of a cDNA for a novel insulin-like peptide of the testicular Leydig cells, J. Biol. Chem., 268: 26668-26672 (1993)). Unter Anwendung der Reversen Transkription - Polymerase-Kettenreaktion (RT-PCR) und nachfolgender Northern-Hybridisierung ist es jedoch gelungen, spezifische Signale für RLF-mRNA auch in Ovarien und Trophoblasten des Menschen nachzuweisen (L. Tashima et al., The human Leydig insulin-like (Ley-I-L) gene is expressed in the corpus luteum and trophoblast, J. Clin. Endocrinol. Metab., 80: 707-710 (1995)).

Die Wiederkäuer nehmen unter den Säugetieren jedoch insofern eine Sonderstellung ein, da sie im Falle der Gravidität zwar deutliche Zeichen einer Relaxin-abhängigen Physiologie aufweisen, bislang aber keine Expression eines Relaxin-Gens nachgewiesen werden konnte (S. Hartung et al., The search for ruminant relaxin, in: A.H. McLennan, G. Tregear und G.D. Bryant-Greenwood (Hrsg.) "Progress in Relaxin Research", Singapore, World scientific Publishing, S. 439 - 456 (1995)). In der Tat ist bei Schafen eine Deletion im Relaxin-Gen festgestellt worden (P.J. Roche et al., A single copy relaxin-like gene sequence is present in sheep, Mol. Cell. Endocrinol., 91: 21-28 (1993)).

Immunologische Nachweise von bovinem Relaxin finden sich in M.J. Fields et al., Evidence for Relaxin in Corpora Lutea of Late Pregnant Cows, Endocrinology 107, N°4, 1980, 869-876, und L.H. Larkin et al., In Vitro Analysis of Antiseror to Relaxin, Acta Endocrinologica 92, N°3, 1979, 568-576.

Da das Gelbkörperhormon Relaxin somit bislang nur bei den nicht-Wiederkäuer-Arten wie Mensch, Pferd, Katze, Schwein, Ratte, Maus, Meerschweinchen und Hamster eindeutig nachgewiesen werden konnte und das Schwangerschafts-abhängige Gonadotropin ausschließlich bei Primaten und Pferden nachweisbar ist, besteht ein erheblicher Bedarf an diagnostischen Möglichkeiten zur Bestimmung der Gravidität bei anderen Säugerarten, wie insbesondere bei Wiederkäuern.

Der vorliegenden Erfindung liegt demgemäß die Aufgabe zugrunde, Diagnosehilfen und Verfahren sowie geeignete Substanzen hierfür bereitzustellen, mit denen eine Gravidität bei Wiederkäuern und insbesondere bei Rindern zuverlässig nachgewiesen werden kann.

Zur Lösung der Aufgabe werden die Diagnosehilfe gemäß Anspruch 1, das Verfahren zum Nachweis der Trächtigkeit von Wiederkäuern gemäß Anspruch 6, die gegen den Relaxin-ähnlichen Faktor von Wiederkäuern gerichteten Antikörper gemäß Anspruch 12 sowie die Verwendung derselben gemäß Anspruch 15 vorgeschlagen. Weitere Ausführungsformen der Diagnosehilfe, des Verfahrens sowie der Antikörper ergeben sich aus den jeweiligen Unteransprüchen.

Im Rahmen der Forschungsanstrengungen, die schließlich zur vorliegenden Erfindung geführt haben, ist es nunmehr gelungen, auch bei weiblichen Wiederkäuern und insbesondere bei Kühen einen Relaxin-ähnlichen Faktor nachzuweisen, der in sehr großen Mengen im Gelbkörper (Corpus luteum) exprimiert wird. Die Expressionsmuster des RLF-Gens von Rindern sind denen des menschlichen Relaxin-Gens mit einem Anstieg in der späten Lutealphase und mit fortsetzender Expression in die Gravidität hinein sehr ähnlich, wodurch die Einsetzbarkeit der erfindungsgemäß vorgeschlagenen Mittel und Verfahren zur Bestimmung der Trächtigkeit von Wiederkäuern und insbesondere von Rindern unterstrichen wird.

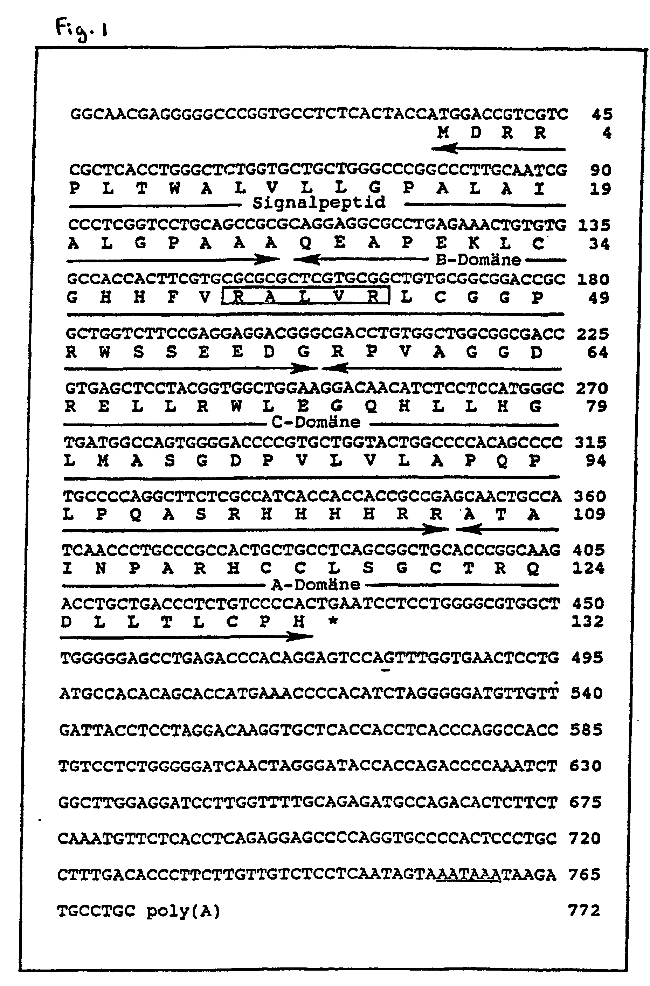

Vom RLF-Protein des Schweines ist bekannt, daß es eine Primärstruktur aufweist, die mit einem A-, B- und C- (connecting) Peptid der Primärstruktur des Insulins ähnlich ist. Alle drei Peptidbereiche sind im RLF-Vorläuferprotein enthalten, welches post-translational in seine Komponenten aufgespaltet wird. Die A- und B-Peptide bilden einen als Heterodimer vorliegenden Faktor, während das C-Peptid vermutlich verworfen wird. Die Primärsequenz des in Figur 1 dargestellten Rinder-RLFs ist in seiner Primärstruktur der des Schweines ähnlich und führt vermutlich gleichermaßen zu einem heterodimeren Faktor. Der RLF-Vorläufer enthält zusätzlich eine Signalsequenz am N-Terminus, die für die kotranslationale Einschleusung des Moleküls in das sekretorische System der Zelle verwendet und nachträglich abgespalten wird. Da diese Struktur auch im RLF-Vorläufer bei Rindern vorhanden ist, ist davon auszugehen, daß auch dieses Protein in die Blutbahn sezerniert wird und demgemäß nachgewiesen werden kann.

Im Rahmen der Erfindung ist mit Hilfe der RT-PCR der gesamte kodierende Bereich des RLF-Gens des Rindes kloniert und sequenziert worden (vgl. Beispiel 1). Ferner wurde mit Hilfe der Northern-RNA-Hybridisierung sowie der RT-PCR eine große Menge von Gewebe des weiblichen und männlichen Rindes hinsichtlich der Expression des Gens untersucht (vgl. Beispiel 2). Die Ergebnisse zeigen, daß der RLF des Rindes ausschließlich in den Leydig-Zellen des Bullen und in den Gelbkörpern der Kuh exprimiert wird. Die in Figur 1 dargestellte Aminosäuresequenz des erfindungsgemäßen Relaxin-ähnlichen Faktors von Wiederkäuern bietet die Möglichkeit, anhand etablierter Verfahren polyklonale und monoklonale Antikörper bzw. Fragmente derselben mit gleicher Immunspezifität zur Verwendung in Diagnosehilfen und immunologischen Nachweisverfahren bereitzustellen. Derartige Antikörper können auf der Basis des vollständigen RLFs sowie auf der Basis von Fragmenten und/oder aktiven Derivaten desselben mit gleicher Immunogenität hergestellt werden.

Der im Rahmen der vorliegenden Beschreibung verwendete Begriff "Antikörper" bezieht sich auf ein Protein, welches aus einem oder mehreren Polypeptiden besteht, die im wesentlichen von Antikörpergenen kodiert werden. Diese Gene schließen verschiedene Gene für konstante Regionen sowie solche für die Vielzahl von variablen Regionen von Antikörpern ein. Die erfindungsgemäßen Antikörper mit Spezifität für den RLF von Wiederkäuern können in. einer Vielzahl von Formen, die insbesondere Fv-, Fab-, Fab'-, F(ab')₂-Fragmente sowie Einzelketten einschließen, solubilisiert oder immobilisiert eingesetzt werden.

Bevorzugte Antikörper sind polyklonale und insbesondere monoklonale Antikörper und Fragmente derselben, die in bezug auf die Interaktion mit dem RLF von Wiederkäuern dieselben Eigenschaften aufweisen. Die Antikörper sind besonders bevorzugt - entweder direkt oder über einen zweiten Immunglobulin-spezifischen Antikörper - mit einem nachweisbaren Marker versehen. Als Marker kommen erfindungsgemäß photoaktivierbare Verbindungen wie Biotin, radioaktive Isotope wie Indium, Jod, Yttrium, Technetium, Rhenium, Kupfer und Lutetium, oder Enzyme wie z.B. Meerrettich-Peroxidase und alkalische Phosphatase in Betracht (vgl. E. Harlow und D. Lane, Antibodies: A Laboratory Manual, Cold Spring Harbor Laboratory Press, Cold Spring Harbor, New York (1988)).

Bei dem erfindungsgemäßen Nachweisverfahren werden dem zu untersuchenden Tier Körperflüssigkeiten oder Gewebe entnommen, in denen der Relaxin-ähnliche Faktor während der Trächtigkeit des Tieres nachweisbar ist, bevor das Vorhandensein des Faktors in der Körperflüssigkeit oder in dem Gewebe des Tieres durch Verwendung der erfindungsgemäßen Antikörper in solubilisierter oder an einen festen Träger immobilisierter Form nachgewiesen wird. Vorzugsweise werden die Körperflüssigkeiten oder Gewebe ausgewählt aus der Gruppe bestehend aus Blut, Plasma, Serum, Urin, Milch und Follikelflüssigkeit.

In besonders bevorzugten Ausführungsformen des erfindungsgemäßen Verfahrens werden die oben genannten spezifischen Antikörper unter Anwendung etablierter Assay-Verfahren nach klassischen Systemen nachgewiesen (vgl. z.B. E. Harlow und D. Lane, a.a.O.).

Die Erfindung wird nachfolgend anhand von Beispielen im einzelnen erläutert.

### Beispiel 1

### Klonierung und Sequenzierung des gesamten kodierenden Bereichs des RLF-Gens des Rindes

Poly(A)⁺ angereicherte RNA wurde hergestellt durch Oligo(dT)-Cellulose-Chromatographie von boviner Gesamt-RNA der Keimdrüsen und als Template zur Herstellung einer cDNA-Bibliothek unter Verwendung des ZAP-cDNA-Synthesekits (Stratagene, La Jolla, CA) eingesetzt. Die cDNA wurde über die EcoRI- und XhoI-Restriktionsstellen in den Vektor Uni-ZAP-XR (Stratagene) kloniert. Die Komplexität der unamplifizierten Bibliothek betrug 1,5 × 10⁶ pfu und der prozentuale Anteil an Nicht-Rekombinanten lag bei weniger als 4 %. Die durchschnittliche Größe der Inserts der cDNA-Klone betrug ca. 1,5 kb innerhalb einer Bandbreite von bis zu > 4 kb.

Anhand der Homologieregion zwischen den veröffentlichten Sequenzen für das RLF-Protein des Schweines und des Menschen (I.M. Adham et al., Coning of a cDNA for a novel insulin-like peptide of the testicular Leydig cells, J. Biol. Chem., 268:26668-26672 (1993); E. Burkhardt et al., A human cDNA coding for the Leydig insulin-like peptide (Ley-I-L), Hum. Genet., 94:91-94 (1994)) wurden zum Erhalt einer spezifischen Sonde zum Screening der cDNA-Bibliothek vorwärts gerichtete und reverse Oligonukleotidprimer hergestellt (vorwärts gerichteter Primer: 5'-CGCTGGTGCGGGTGTGCGG-3'; reverser Primer: 5'-GTCTTGCTGGGTGCAGCC-3'). Diese Primer wurden anschließend in einer RT-PCR eingesetzt, wobei 1 µl der zur Herstellung der obigen cDNA-Bibliothek verwendeten Reaktionsansatzes zur Erststrangsynthese als Template eingesetzt wurde. Das resultierende PCR-Produkt von 254 bp wurde in den Vektor pGEM T (Promega; Madison, WI) kloniert und sequenziert. Dieses PCR-Produkt wurde anschließend mit [α³²P]dCTP markiert und zum Screening von ca. 2500 pfu der bovinen Testis-cDNA-Bibliothek unter Anwendung üblicher Hybridisierungsverfahren eingesetzt. Durch doppelsträngige DNA-Sequenzierung wurden drei unabhängige cDNA-Klone mit den längsten cDNA-Inserts identifiziert. Da keine der erhaltenen Sequenzen das vollständige 5'-Ende des RLF-Transkripts umfaßte, wurde eine PCR-Strategie angewendet, wobei als Stromaufwärts-Primer die T3-Polymerasebindende Stelle in dem pBluescript-Vektor der cDNA-Bibliothek und als Stromabwärts-Primer ein internes bovines RLF-spezifisches Oligonukleotid (5'-CCGCCAGCCACAGGTCGC-3') sowie 5 µl (ca. 10 000 pfu) der unamplifizierten cDNA-Bibliothek als Template eingesetzt wurden. Die PCR-Bedingungen für insgesamt 30 Zyklen waren wie folgt: Anelierung bei 65 °C, 1 min; Elongation bei 72 °C, 1 min; Denaturierung bei 95 °C, 1 min. Die Produkte wurden dann einer ebenfalls 30 Zyklen umfassenden PCR zugeführt, wobei derselbe Stromabwärts-Primer sowie ein neuer, interner Stromaufwärts-Primer (5'-CGAGCGCGCGCACGAAGTGG-3') eingesetzt wurden. Die Endprodukte wurden auf einem 3%igen Agarosegel elektrophoretisch aufgetrennt, und die größten der erhaltenen DNA-Fragmente wurden in den Vektor pGEM T (Promega) subkloniert und sequenziert. Diese Reaktionen wurden für drei unabhängige Aliquots der cDNA-Bibliothek wiederholt.

Um die Transkripte zu charakterisieren, die gegenüber dem bovinen Gelbkörper positive Hybridisierungssignale zeigten, wurde luteale RNA aus einer späten Phase der Gravidität verwendet, um eine RT-PCR durchzuführen, wie sie bereits oben bezüglich der Testis-RNA beschrieben wurde. Das resultierende PCR-Fragment wurde wie oben beschrieben kloniert und sequenziert.

Die Anzahl positiver Klone beim ursprünglichen Screening der bovinen Testis-cDNA-Bibliothek ergab eine sehr hohe Transkripthäufigkeit von 0,7 %.

Die anhand von drei unabhängigen cDNA-Klonen und RT-PCR-Produkten bestimmte und bestätigte cDNA-Sequenz vollständiger Länge ist in Figur 1 dargestellt. Das Transkript von 790 Basenpaaren kodiert für ein Polypeptid von 132 Aminosäuren mit Homologien von 52 %, 70 % und 87 % gegenüber den entsprechenden RLF- bzw. LEY-I-L-Aminosäuresequenzen der Maus, des Menschen und des Schweines. Der N-Terminus der Protein-kodierenden Region verfügt über sämtliche Merkmale eines Signalpeptids und wird höchstwahrscheinlich nach dem Alaninrest an der Position 26 abgespalten. Die jeweiligen Bereiche für die A-, B- und C-Domänen sowie für das Rezeptor-Bindungsmotiv und das 3'-Polyadenylierungssignal sind hervorgehoben.

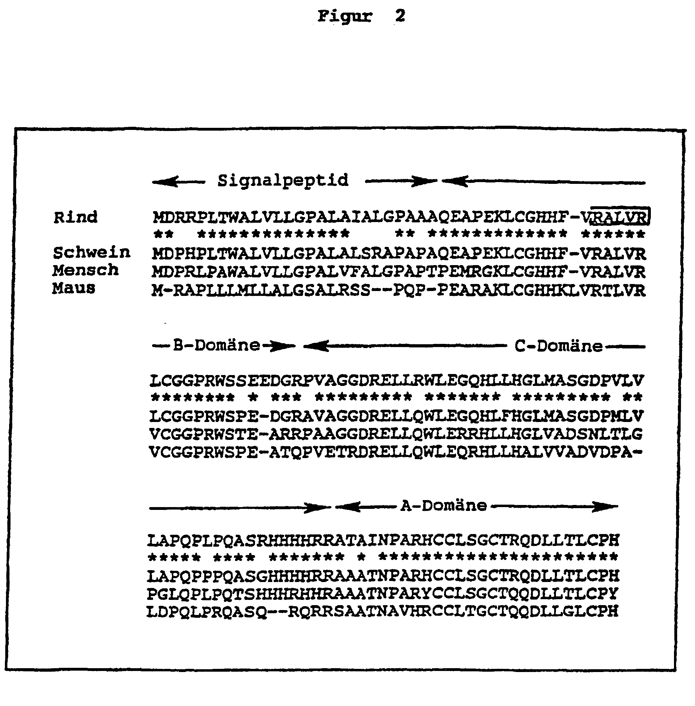

Die Figur 2 zeigt einen Homologievergleich zwischen der bovinen RLF-Sequenz und denjenigen der bekannten Relaxin-Moleküle vom Schwein, vom Menschen und von der Maus. Sowohl das Muster der konservierten Cysteinreste als auch die putative Rezeptor-Bindungssequenz -R-A-L-V-R- sowie die hohe Konservierung der A-B-Insulin-ähnlichen Peptiddomäne zeigen Ähnlichkeiten. Obgleich die Regionen, die mit den bekannten Spaltstellen im Relaxin-Vorläufer des Schweines korrespondieren, auch konserviert zu sein scheinen, gibt *es* noch keine Beweise dafür, daß das RLF-Molekül tatsächlich in seine A-, B- und C-Bestandteile gespalten wird.

### Beispiel 2

### RNA-Analyse unterschiedlicher Gewebe

Verschiedene Gewebe des weiblichen Rindes wurden so ausgewählt, daß sie unterschiedliche Stadien des Östrogenzyklus und der Gravidität abdeckten. Die Präparation der RNA erfolgte nach herkömmlichen Verfahren durch Extraktion mit Guanidiniumisothiocyanat und Ultrazentrifugation durch ein CsCl-Kissen. Die Präparation der RNA aus dem von 3jährigen Bullen gewonnenen Keimdrüsenmaterial erfolgte wie oben beschrieben. Nach elektrophoretischer Auftrennung der jeweiligen Gesamt-RNA sowie nach Übertragung auf Nylonmembranen wurden Northern-Blots hergestellt und mit dem radioaktiv markierten, spezifischen 254 bp umfassenden DNA-Fragment der bovinen RLF-cDNA (siehe Beispiel 1) hybridisiert. Für die in situ-Hybridisierung wurde zusätzliches bovines und ovines testikuläres Gewebe von geschlechtsreifen Tieren gesammelt und zur nachfolgend beschriebenen Bouin-Fixierung und Paraffineinbettung vorbereitet.

Für Uterus- und andere Gewebe, die bei der Northern-Hybridisierung keine oder lediglich schwache Signale zeigten, wurde ein RT-PCR-Assay geschaffen unter Verwendung spezifischer, von der bovinen cDNA-Sequenz gemäß Figur 1 abgeleiteter vorwärts gerichteter und reverser Primer (vorwärts gerichteter Primer: 5'-CGCGCTGGTCTTCCGAGG-3'; reverser Primer: 5'-GTCTTGCTGGGTGCAGCC-3'), wodurch ein Produkt von 209 bp erhalten wurde. Die Primer wurden so geschaffen, daß sie die einzige für das humane Gen definierte Spleißstelle (E. Burkhardt et al., A Human cDNA coding for the Leydig insulin-like peptide (Ley-I-L), Hum. Genet., 94:91-94 (1994)) abdeckten, damit eine mögliche Kontaminierung der cDNA-Templates durch genomische DNA vermieden wurde. Die PCR-Bedingungen waren wie in Beispiel 1 beschrieben. Nach elektrophoretischer Auftrennung der PCR-Produkte in Agarosegelen wurden diese auf Nylonmembranen (Hybond N; Amersham-Buchler) übertragen und gegen ein internes, radioaktives und für die bovine RLF-cDNA-Sequenz spezifisches Oligonukleotid (5'-GGCCCCA-CAGCCCCTGCCCCAGG-3') hybridisiert. Als Kontrolle für die Qualität der hergestellten cDNA erfolgten parallele PCR-Reaktionen unter Verwendung von Primern, die für die bovine GAPDH-mRNA spezifisch sind (R. Ivell et al., Oxytocin and oxytocin receptor gene expression in the reproductive tract of the pregnant cow: rescue of luteal oxytocin production at term, Biol. Reprod., 53:553-560 (1995)).

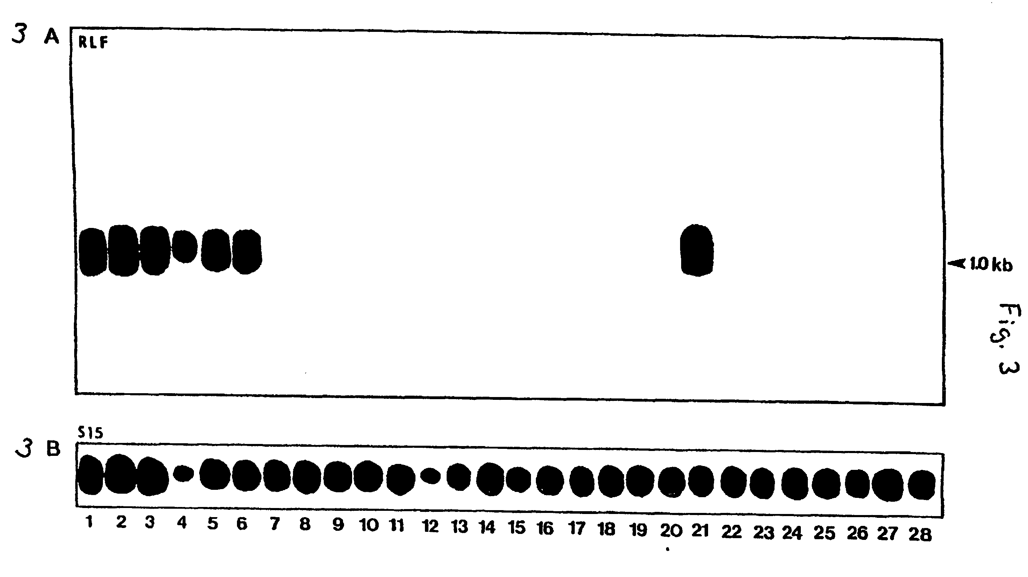

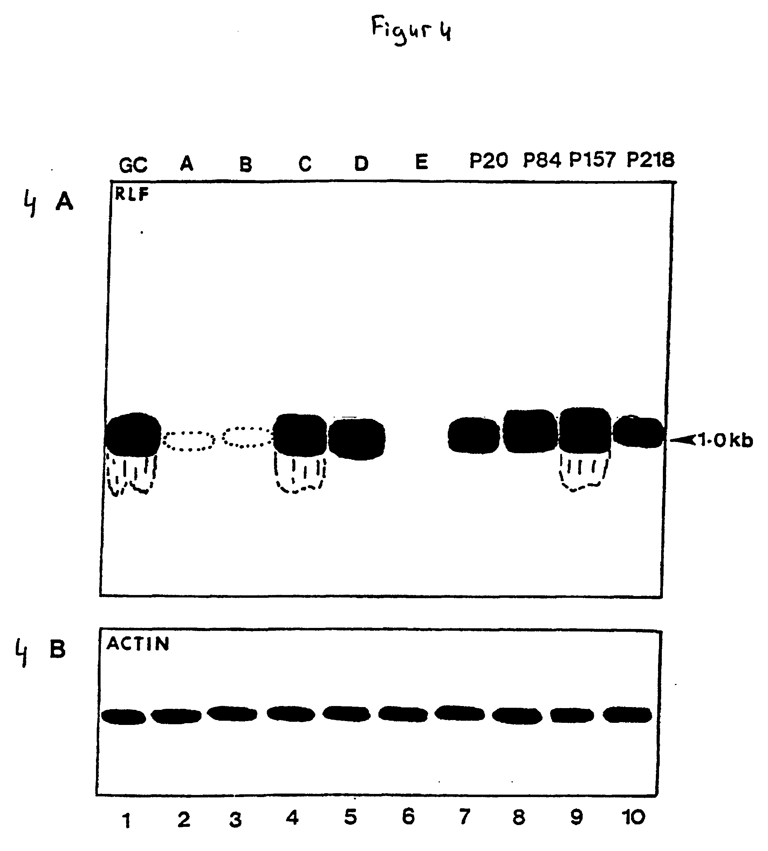

Eine Northern-Hybridisierung von Gesamt-RNA verschiedener boviner Gewebe sowohl von männlichen als auch von weiblichen Rindern zeigte wie erwartet ein starkes Signal im Testis (Figur 3). Zusätzlich wurden deutliche Signale jedoch auch im Corpus luteum, der follikulären Granulosa und den Theka-Zellen sowie in ovariellem Stromagewebe aus dem Östrogenzyklus beobachtet. Andere Gewebe einschließlich des Endometriums und Myometriums sowohl aus dem Stadium des Zyklus als auch aus der Gravidität sowie des Eileiters, der Pinealdrüse, der Leber, des Herzens, der Lunge, der Milz, der Nebennierendrüse, des Epididymis, der Prostata, der Schilddrüse, der Hypophyse, des Cerebellums und der Großhirnrinde waren negativ (Figur 3). Die Northern-Hybridisierung sämtlicher positiver Gewebe wies auf eine mRNA in einer Größe von ca. 1,0 kb hin, was einem Transkript vollständiger Länge von 790 Basen plus etwa 200 Basen Poly(A)-Schwanz entspricht. Die Ergebnisse der Northern-Hybridisierungen von Gesamt-RNA aus verschiedenen bovinen Geweben unter Verwendung des 254 bp umfassenden Fragments als Sonde sind in Figur 3 dargestellt (Bild A). Als Kontrolle wurde der Blot mit einem für das ribosomale Protein S15 spezifischen bovinen cDNA-Fragment rehybridisiert (Bild B). Die Belegung der Spuren erfolgte'mit RNA der folgenden Gewebetypen:
1. Ovariales Stroma
2. Theka-Zellen
3. Granulosa-Zellen
4. Corpus luteum der mittleren Graviditätsphase (Tag 150)
5. Corpus luteum der späten Zyklusphase (ca. Tag 17)
6. Corpus luteum der mittleren Zyklusphase (Tag 7)
7. Myometrium der späten Graviditätsphase (Tag 280)
8. Myometrium der mittleren Graviditätsphase (Tag 150)
9. Myometrium der mittleren Zyklusphase (Tag 7)
10. Myometrium des Östrus (Tag 0)
11. Endometrium der späten Graviditätsphase (Tag 280)
12. Endometrium der mittleren Graviditätsphase (Tag 150)
13. Endometrium der mittleren Zyklusphase (Tag 7)
14. Endometrium des Östrus (Tag 0)
15. Großhirnrinde
16. Cerebellum
17. Hypophyse
18. Schilddrüse
19. Prostata
20. Epididymis
21. Testis
22. Nebenniere
23. Milz
24. Lunge
25. Herz
26. Leber
27. Eileiter
28. Pinealdrüse.

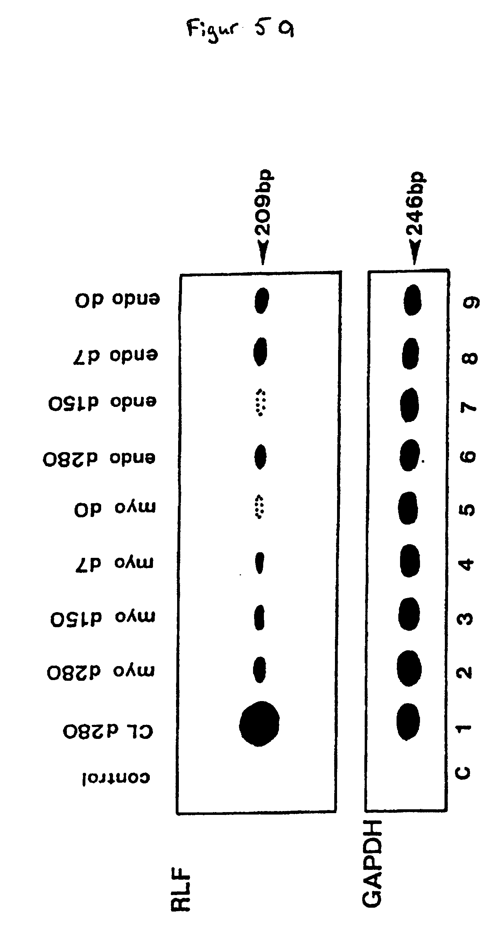

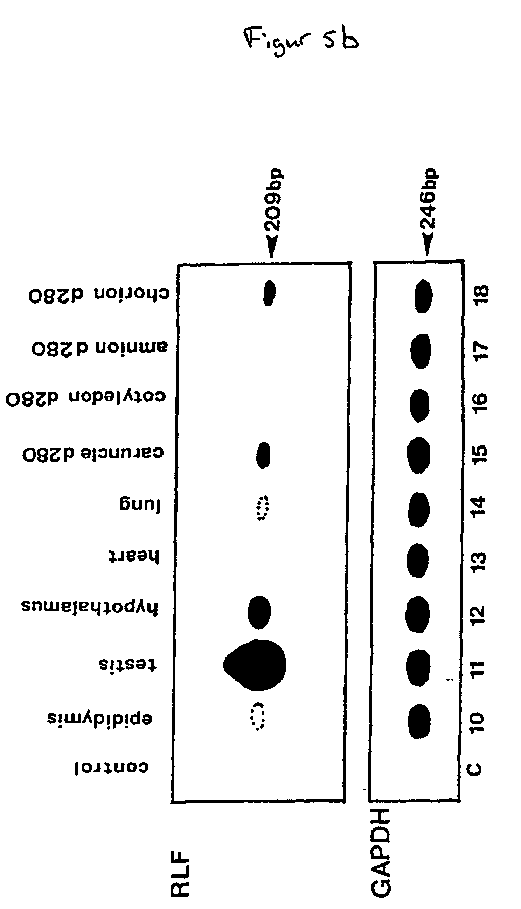

Die Northern-Hybridisierung von Gesamt-RNA aus Gelbkörpern unterschiedlicher Stadien des Östrogenzyklus und der Gravidität (Figur 4) bestätigt die relativ hohe Expression in aus prä-ovulatorischen Follikeln frisch präparierten Granulosa-Zellen. Innerhalb des Gelbkörpers kommt es in der zweiten Hälfte des Östrogenzyklus zu einem deutlichen Anstieg der spezifischen RLF-mRNA, wobei maximale Werte in der mittleren Graviditätsphase erreicht werden. In der späten Graviditätsphase scheint die RLF-mRNA reduziert zu sein. Im Corpus albicans ist die RLF-mRNA selbst nach ausgedehnter Expositionszeit nicht nachweisbar. Die Ergebnisse aus der Northern-Hybridisierung unter Verwendung einer spezifischen bovinen RLF-cDNA-Sonde von Gesamt-RNA aus Granulosa-Zellen (GC) und Gelbkörpern aus unterschiedlichen Stadien des Zyklus und der Gravidität sind in Figur 4A dargestellt. Zur Kontrolle der gleichmäßigen Beladung mit RNA wurde der Blot mit einer Actin-Sonde (Bild B) rehybridisiert. Die in Figur 4 verwendeten Bezeichnungen haben die folgenden Bedeutungen:
- A =: Tage 2-3 der frühen Zyklusphase
- B =: Tage 4-6 der frühen mittleren Zyklusphase
- C =: Tage 8-14 der mittleren Zyklusphase
- D =: Tage 15-18 der späten Zyklusphase
- E =: Corpus albicans
- P =: Gravidität, wobei die Ziffern die Tage der Gravidität angeben.

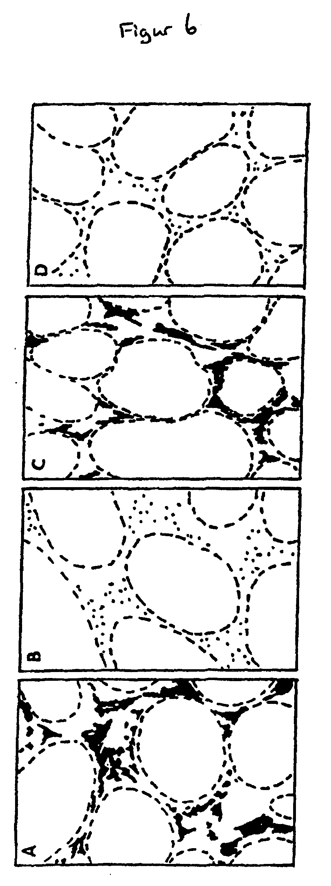

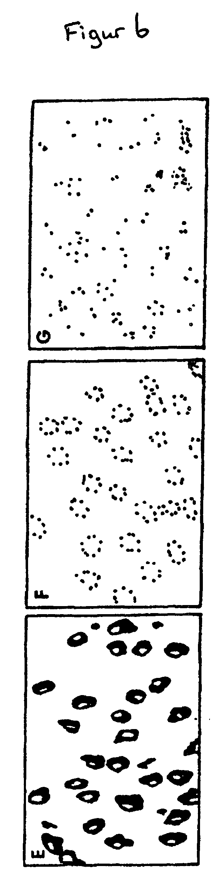

Eine RT-PCR-Analyse wurde angewendet, um andere bovine Gewebe wie insbesondere solche zu untersuchen, die während der Gravidität mit Relaxin-bedingter Physiologie assoziiert sind. In Figur 5 sind die Ergebnisse der RT-PCR-Analysen für RLF- und GAPDH-Gentranskripte in RNA-Proben aus den angegebenen Geweben dargestellt. Die Bezeichnungen der Spuren haben die folgenden Bedeutungen:
- C =: Kontrolle
- 1 =: Corpus luteum der späten Graviditätsphase (Tag 280)
- 2 =: Myometrium der späten Graviditätsphase (Tag 280)
- 3 =: Myometrium der mittleren Graviditätsphase (Tag 150)
- 4 =: Myometrium der mittleren Zyklusphase (Tag 7)
- 5 =: Myometrium des Östrus (Tag 0)
- 6 =: Endometrium der späten Graviditätsphase (Tag 280)
- 7 =: Endometrium der mittleren Graviditätsphase (Tag 150)
- 8 =: Endometrium der mittleren Zyklusphase (Tag 7)
- 9 =: Endometrium des Östrus (Tag 0)
- 10 =: Epididymis
- 11 =: Testis
- 12 =: Hypothalamus
- 13 =: Herz
- 14 =: Lunge
- 15 =: Karunkel der späten Graviditätsphase (Tag 280)
- 16 =: Plazentalappen der späten Graviditätsphase (Tag 280)
- 17 =: Amnion der späten Graviditätsphase (Tag 280)
- 18 =: Chorion der späten Graviditätsphase (Tag 280)

Die Kontrollen zeigen parallele Reaktionen, in denen RNA durch Wasser ersetzt wurde.

Obgleich die der Figur 5 zugrundeliegende Technik lediglich semi-quantitativ ist, gibt es in den meisten Geweben sehr schwache positive Signale. Wie erwartet, ergaben die als positive Kontrollen eingesetzten Proben aus bovinem Corpus luteum und Testis sehr starke Signale. Interessanterweise wurde auch beim bovinen Hypothalamus ein Signal mittlerer Stärke beobachtet.

### In situ-Transkript-Hybridisierung

Testis- und Ovarialfragmente wurden 6 Stunden lang in Bouin's-Lösung fixiert, in 70 % Ethanol gewaschen und in Paraffinwachs eingebettet. Schnitte mit einer Dicke von 10 µm wurden anschließend einer nicht-radioaktiven Hybridisierung im wesentlichen nach Maguire et al. (S.M. Maguire et al., Stage-dependent expression of mRNA for cyclic protein 2 during spermatogenesis is modulated by elongate spermatids, Mol. Cell. Endocrinol., 94:79-88 (1993)) zugeführt, wobei durch in vitro-Transkription des ursprünglichen, mittels PCR klonierten Produkts (s.o.) markierte cRNA-Sonden in Gegenwart von Digoxigenin-UTP (Boehringer-Mannheim, Mannheim, BRD) eingesetzt wurden. Die negativen Kontrollen erfolgten parallel unter Verwendung der Sense-Strang-cRNA. Die Schnitte wurden leicht mit Methylgrün gegengefärbt.

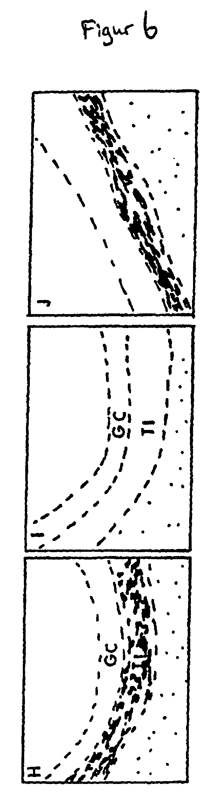

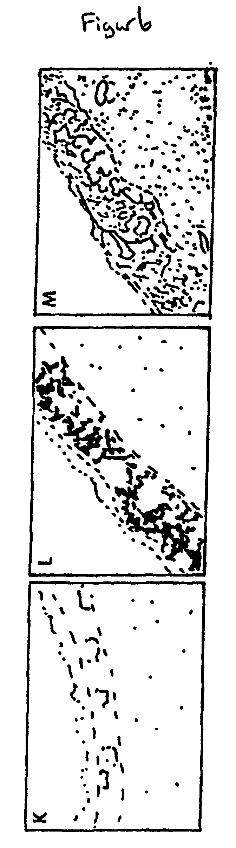

Die entsprechenden Ergebnisse sind in Figur 6 dargestellt.

Die in der Figur 6 verwendeten Buchstaben haben die folgende Bedeutung:
- A -: Adulter boviner Testis (Anti-Sense-Sonde, X 200).
- B -: Adulter boviner Testis (Sense-Sonde als Kontrolle, X 200).
- C -: Adulter oviner Testis (Anti-Sense-Sonde, X 200).
- D -: Adulter oviner Testis (Sense-Sonde als Kontrolle, X 200).
- E -: Corpus luteum (mittlere - späte Zyklusphase; Anti-Sense-Sonde, X 400).
- F -: Corpus luteum (mittlere - späte Zyklusphase; Sense-Sonde als Kontrolle, X 400).
- G -: Corpus luteum (späte Zyklusphase; Anti-Sense-Sonde, X 200).
- H -: Gesunder Antral-Follikel (frühe Zyklusphase; Anti-Sense-Sonde, X 200; GC, Granulosa-Zellschicht; TI, Theca interna.
- I -: Wie in H, aber unter Verwendung von Sense-Sonde als Kontrolle.
- J -: Gesunder präovulatorischer Follikel (späte Zyklusphase; Anti-Sense-Sonde, X 200).
- K -: Atretischer Follikel (mittlere Zyklusphase; Sense-Sonde als Kontrolle; X 200).
- L -: Wie in K, aber unter Verwendung einer Anti-Sense-Sonde.
- M -: Wie in K, aber histologisch nur mit Hämotoxilin-Eosin gefärbt.

Die Schnitte des in E - G gezeigten Corpus luteum erforderten zur Entfernung unspezifischen Hintergrundes ein wesentlich stringenteres Waschen, was gegenüber den anderen Schnitten zu schwächeren spezifischen Signalen mit der Anti-Sense-Sonde führte.

Die Ergebnisse zeigen, daß bei sowohl bovinen als auch ovinen Testis (Figur 6A und C) die RLF-Gentranskripte ausschließlich in den Leydig-Zellen nachgewiesen werden, wie es auch bei anderen Spezies gezeigt worden ist. Innerhalb des Ovars (Figur 6E - M) erscheint ein stark positives Signal in der Theka-Zellschicht großer antraler Follikel aus der frühen und späten Zyklusphase (Figur 6H und J) wie auch im Corpus luteum der mittleren bis späten Zyklusphase (Figur 6E). Sowohl Stromazellen als auch follikuläre Granulosa-Zellen scheinen unter Anwendung dieser Technik negativ zu sein und heben sich demgemäß von den negativen Kontrollschnitten unter Verwendung der Sense-cRNA-Sonde (Figur 6F, I und K) nicht ab.

## Patentansprüche

1. Diagnosehilfe zum Nachweis der Trächtigkeit von Wiederkäuern, **dadurch gekennzeichnet, daß** sie Antikörper gegen den Relaxin-ähnlichen Faktor von Wiederkäuern bzw. Fragmente derselben mit gleicher Immunspezifität enthält.

2. Diagnosehilfe nach Anspruch 1, **dadurch gekennzeichnet, daß** die Wiederkäuer ausgewählt sind aus der Gruppe bestehend aus Rindern, Ziegen, Schafen, Kamelen, Dromedaren.

3. Diagnosehilfe nach Anspruch 1 oder 2, **dadurch gekennzeichnet, daß** der Wiederkäuer ein Rind ist.

4. Diagnosehilfe nach einem der vorhergehenden Ansprüche, **dadurch gekennzeichnet, daß** die Antikörper gegen den Relaxin-ähnlichen Faktor von Wiederkäuern als solchen oder gegen Fragmente und/oder aktive Derivate desselben mit gleicher Immunogenität gerichtet sind.

5. Diagnosehilfe nach einem der vorhergehenden Ansprüche, **dadurch gekennzeichnet, daß** die Antikörper monoklonale Antikörper sind.

6. Verfahren zum Nachweis der Trächtigkeit von Wiederkäuern, **dadurch gekennzeichnet, dass** man das Vorhandensein des Relaxin-ähnlichen Faktors in einer Körperflüssigkeits- oder Gewebsprobe des zu untersuchenden Tiers nachweist, in der der Relaxin-ähnliche Faktor während der Trächtigkeit des Tieres nachweisbar ist.

7. Verfahren nach Anspruch 6, **dadurch gekennzeichnet, daß** die Wiederkäuer ausgewählt werden aus der Gruppe bestehend aus Rindern, Ziegen, Schafen, Kamelen, Dromedaren.

8. Verfahren nach Anspruch 6 oder 7, **dadurch gekennzeichnet, daß** der Wiederkäuer ein Rind ist.

9. Verfahren nach einem der Ansprüche 6 bis 8, **dadurch gekennzeichnet, daß** die Körperflüssigkeiten oder Gewebe ausgewählt werden aus der Gruppe bestehend aus Blut, Plasma, Serum, Urin, Milch und Follikelflüssigkeit.

10. Verfahren nach einem der Ansprüche 6 bis 9, **dadurch gekennzeichnet, daß** das Vorhandensein des Relaxin-ähnlichen Faktors durch Anwendung eines Radioimmunoassays nachgewiesen wird.

11. Verfahren nach einem der Ansprüche 6 bis 9, **dadurch gekennzeichnet, daß** das Vorhandensein des Relaxin-ähnlichen Faktors durch Anwendung eines Enzym-gekoppelten Immunoassays nachgewiesen wird.

12. Antikörper und Antikörperfragmente gegen den Relaxin-ähnlichen Faktor von Wiederkäuern als solchen oder gegen Fragmente und/oder aktive Derivate desselben mit gleicher Immunogenität.

13. Antikörper nach Anspruch 12, **dadurch gekennzeichnet, daß** sie monoklonale Antikörper sind.

14. Antikörper nach Anspruch 12 oder 13, **dadurch gekennzeichnet, daß** sie mit einem nachweisbaren Marker versehen sind.

15. Verwendung der Antikörper gemäß einem der Ansprüche 12 bis 14 zum Nachweis der Trächtigkeit von Wiederkäuern.

## Claims

1. Diagnosis aid for detecting pregnancy in ruminants, **characterised in that** it contains antibodies against the relaxin-like factor of ruminants or fragments of the same with the same immunospecificity.

2. Diagnosis aid as claimed in Claim 1, **characterised in that** the ruminants are chosen from the group consisting of cattle, goats, sheep, camels, dromedaries.

3. Diagnosis aid as claimed in Claim 1 or 2, **characterised in that** the ruminants are cattle.

4. Diagnosis aid as claimed in one of the preceding claims, **characterised in that** the antibodies are directed against the relaxin-like factor of ruminants as such or against fragments and/or active derivatives of the same with the same immunogenicity.

5. Diagnosis aid as claimed in one of the preceding claims, **characterised in that** the antibodies are monoclonal antibodies.

6. Process for detecting pregnancy in ruminants, **characterised in that** the presence of the relaxin-like factor is detected in a body fluid or tissue sample of an animal to be examined, in which the relaxin-like factor is detectable during the pregnancy of the animal.

7. Process as claimed in Claim 6, **characterised in that** the ruminants are chosen from the group consisting of cattle, goats, sheep, camels, dromedaries.

8. Process as claimed in Claim 6 or 7, **characterised in that** the ruminants are cattle.

9. Process as claimed in one of Claims 6 to 8, **characterised in that** the body fluids or tissue are chosen from the group consisting of blood, plasma, serum, urine, milk and follicle fluid.

10. Process as claimed in one of Claims 6 to 9, **characterised in that** the presence of the relaxin-like factor is detected by using a radioimmunoassay.

11. Process as claimed in one of Claims 6 to 9, **characterised in that** the presence of the relaxin-like factor is detected by using an enzyme-coupled immunoassay.

12. Antibodies and antibody fragments against the relaxin-like factor of ruminants as such or against fragments and/or active derivatives of the same with the same immunogenicity.

13. Antibodies as claimed in Claim 12, **characterised in that** they are monoclonal antibodies.

14. Antibodies as claimed in Claim 12 or 13, **characterised in that** they are provided with a detectable marker.

15. Use of the antibodies as claimed in one of Claims 12 to 14 for detecting pregnancy in ruminants.

## Revendications

1. Auxiliaire de diagnostic pour la détermination de la gravidité de ruminants, **caractérisé en ce qu'**il contient des anticorps dirigés contre le facteur ressemblant à la relaxine de ruminants ou des fragments de celui-ci ayant la même immunospécificité.

2. Auxiliaire de diagnostic selon la revendication 1, **caractérisé en ce que** les ruminants sont choisis dans le groupe constitué par les bovins, les caprins, les ovins, les chameaux, les dromadaires.

3. Auxiliaire de diagnostic selon la revendication 1 ou 2, **caractérisé en ce que** le ruminant est un bovin.

4. Auxiliaire de diagnostic selon l'une quelconque des revendications précédentes, **caractérisé en ce que** les anticorps sont dirigés contre le facteur ressemblant à la relaxine de ruminants tel quel ou contre des fragments et/ou des dérivés actifs de celui-ci ayant la même immunogénicité.

5. Auxiliaire de diagnostic selon l'une quelconque des revendications précédentes, **caractérisé en ce que** les anticorps sont des anticorps monoclonaux.

6. Procédé pour la détermination de la gravidité de ruminants, **caractérisé en ce qu'**on détecte la présence du facteur ressemblant à la relaxine dans un échantillon de liquide corporel ou de tissu de l'animal à examiner, dans lequel le facteur ressemblant à la relaxine est détectable pendant l'état de gravidité de l'animal.

7. Procédé selon la revendication 6, **caractérisé en ce que** les ruminants sont choisis dans le groupe constitué par les bovins, les caprins, les ovins, les chameaux, les dromadaires.

8. Procédé selon la revendication 6 ou 7, **caractérisé en ce que** le ruminant est un bovin.

9. Procédé selon l'un quelconque des revendications 6 à 8, **caractérisé en ce que** les liquides corporels ou les tissus sont choisis dans le groupe constitué par le sang, le plasma, le sérum, l'urine, le lait et le liquide folliculaire.

10. Procédé selon l'une quelconque des revendications 6 à 9, **caractérisé en ce que** la présence du facteur ressemblant à la relaxine est détectée par utilisation d'un dosage radio-immunologique.

11. Procédé selon l'une quelconque des revendications 6 à 9, **caractérisé en ce que** la présence du facteur ressemblant à la relaxine est détectée par utilisation d'un dosage immunologique couplé à une enzyme.

12. Anticorps et fragments d'anticorps dirigés contre le facteur ressemblant à la relaxine de ruminants tel quel ou contre des fragments et/ou des dérivés actifs de celui-ci ayant la même immunogénicité.

13. Anticorps selon la revendication 12, **caractérisés en ce qu'**il s'agit d'anticorps monoclonaux.

14. Anticorps selon la revendication 12 ou 13, **caractérisés en ce qu'**ils sont munis d'un marqueur détectable.

15. Utilisation des anticorps selon l'une quelconque des revendications 12 à 14, pour la détermination de la gravidité de ruminants.
